(19) Europäisches Patentamt
European Patent Office
Office européen des brevets

(11) Publication number : **0 600 782 A1**

(12) **EUROPEAN PATENT APPLICATION**

(21) Application number : **93402886.1**

(51) Int. Cl.$^5$ : **C07H 15/244,** A61K 31/71

(22) Date of filing : **29.11.93**

(30) Priority : **30.11.92 US 983004**

(43) Date of publication of application : **08.06.94 Bulletin 94/23**

(84) Designated Contracting States : **AT BE CH DE DK ES FR GB GR IE IT LI LU MC NL PT SE**

(71) Applicant : **Bristol-Myers Squibb Company**
**345 Park Avenue**
**New York, N.Y. 10154 (US)**

(72) Inventor : **Hoshi, Hideaki**
**4-20-12 Fukuei**
**Ichikawa (JP)**
Inventor : **Aburaki, Shimpei**
**4-24-1-302 Ikuta**
**Tama-ku, KLawasaki (JP)**

(74) Representative : **Durand, Yves Armand Louis**
**Cabinet Z. Weinstein**
**20, Avenue de Friedland**
**F-75008 Paris (FR)**

(54) **C-11 modified pradimicin derivatives.**

(57) A new group of pradimicin derivatives having substituents other than $OCH_3$ in the C-11 position have been discovered. They exhibit antibiotic activity. The compounds, and their production from OH- or $OCH_3$-substituted analogs, are described.

EP 0 600 782 A1

Background

The search for antifungal antibiotics has led to the discovery of the pradimicins. Derived from naturally occurring organisms, such as actinomycetes, the pradimicins protect mice from lethal systemic infections such as Candida albicans, Cryptococcus neoformans and Aspergillus fumigatus.

U.S. patents 4,870,165, 4,973,673, 4,990,497, 4,992,425, 5,053,395 and 5,091,418 disclose pradimicins which are, respectively, antifungal agents and alpha-glucosidase inhibitors.

One group of known pradimicins is covered by formula A:

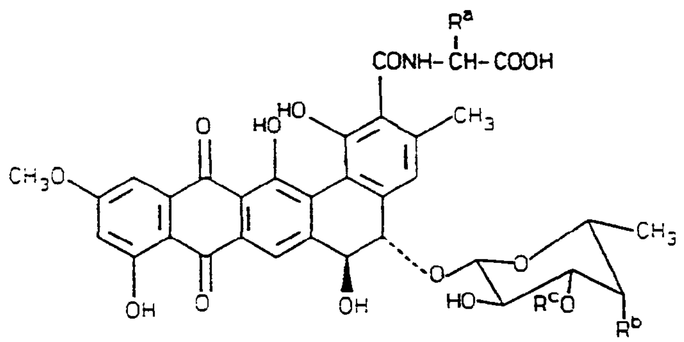

(A)

wherein $R^a$ is H, $CH_3$, or $CH_2OH$;
$R^b$ is $NH_2$, OH or $NHCH_3$; and
$R^c$ is H or β-D-xylose.

All of these compounds have a methoxy, or $OCH_3$, group in the C-11 position.

Recently, new substituted pradimicins containing 11-0-xylosyl groups, know as pradimicins T1, T2 and 11-0-dexylosylpradimicin T1 were discovered. T1 and T2 are produced by fermentation of actinomycete strain AA3798 (a culture of which is on deposit pursuant to the Budapest Treaty as ATCC No. 55288). The third compound is derived from T1.

Figure B covers these xylosyl-substituted pradimicins:

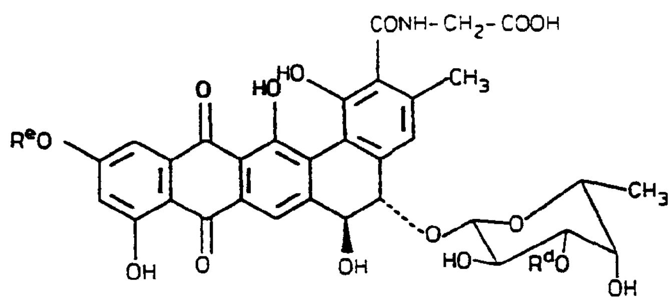

(B)

wherein $R^d$ is H or β-D-xylose and $R^e$ is H or

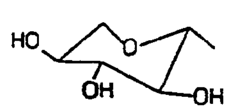

(i.e., β-L-xylose).

In pradimicin T1, $R^d$ is β-D-xylose and $R^e$ is β-L-xylose. In pradimicin T2, $R^d$ is H and $R^e$ is β-L-xylose. In 11-0-dexylosyl-pramidicin T1, $R^d$ is β-D-xylose and $R^e$ is H.

The Invention

It has been discovered that new biologically active pradimicin derivatives can be made from xylosyl pradimicins. The compounds of the invention do not have methoxy or xylosyl groups at the C-11 position of the pradimicin molecule. They are devoid of methoxy and xylosyl moieties at the 11-position. They are chemically distinct from other known antifungal antibiotics.

## Detailed Description of the Invention

Unless otherwise stated, all percentages recited are weight percentages, based on total composition weight.

The invention deals with the new compounds, methods for their preparation and processes and compositions which use them.

The pradimicins described herein have biological activity similar to that of the xylosyl pradimicins referred to herein.

## The Compounds

The compounds of the invention conform to structure I:

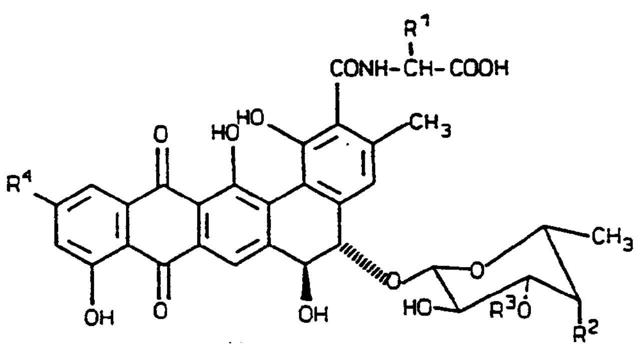

(I)

wherein

$R^1$ is H, $CH_3$, or $CH_2OH$;

$R^2$ is OH or $NR^5R^6$;

$R^3$ is H or

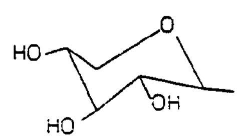

(β-D-xylose);

$R^5$ and $R^6$ are H or $CH_3$;

$R^4$ is H, OH, $OR^7$, $OCH_2R^8$, $0S0_2CF_3$ $N(CH_3)_2$, $CONH_2$, or CN;

$R^7$ is $C_{2-5}$ alkyl, $C_{2-5}$ alkenyl or $C_{2-5}$ haloalkyl (halo = Cl or F); and

$R^8$ is phenyl, COOH or $CONH_2$, with the proviso that when $R^4$ is OH, $R^1$ is not H.

One preferred group of compounds are listed in Table I, along with the appropriate designations for $R^1$, $R^2$, and $R^3$, $R^4$ is always OH in compounds $I_{A-F}$.

Thus, these compounds are of Formula I-I:

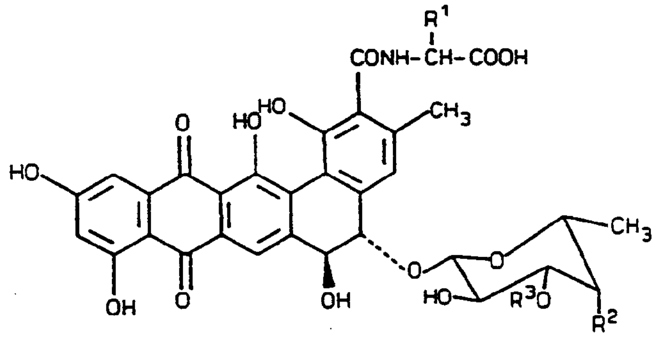

(I - I)

wherein $R^1$, $R^2$ and $R^3$ have the meanings given in Table I.

| Table I: Compounds $I_{A-F}$* | | | |
|---|---|---|---|
| Compound | $R^1$ | $R^2$ | $R^3$ |
| $I_A$ | $CH_3$ | $NHCH_3$ | ß-D-Xylose |
| $I_B$ | $CH_3$ | $NHCH_3$ | H |
| $I_C$ | $CH_3$ | $NH_2$ | ß-D-Xylose |
| $I_D$ | $CH_2OH$ | $NHCH_3$ | ß-D-Xylose |
| $I_E$ | $CH_2OH$ | $N(CH_3)_2$ | ß-D-Xylose |
| $I_F$ | $CH_2OH$ | OH | ß-D-Xylose |
| *$R^4$=OH | | | |

The compounds in Table I were prepared by treating the 11-OMe-pradimicins (i.e., 11-methoxypradimicins) with an aryl ether cleavage reagent, such as LiI (I.T. Harrison et al., Chem. Commun., 616, 1969) in an appropriate organic solvent such as collidine, lutidine or dimethylformamide, under heating at about 160-200°C for a reaction period of about 30 minutes (1/2 hour) to about 16 hours, in the presence or absence of acid scavenger, such as molecular sieve 4A. The products were purified on a $C_{18}$ reversed phase column, eluting with 10-30% acetonitrile-phosphate buffer (pH 3.5).

Another useful group of pradimicin derivatives are those covered by formula II:

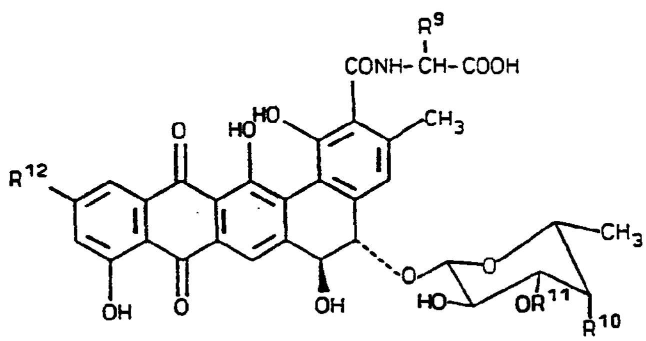

(II)

wherein

$R^9$ is H or $CH_3$;

$R^{10}$ is OH or $NHCH_3$;

$R^{11}$ is H or β-D-xylose; and

$R^{12}$ is H, $OC_2H_5$, $OCH_2CH_2F$, $OCH_2CH=CH_2$, $OCH_2$phenyl ($OCH_2Ph$), $OCH_2COOH$, $OCH_2CONH_2$,

$N(CH_3)_2$, $CONH_2$, or CN.

These derivatives are obtained from 11-hydroxy pradimicins by alkylation or via the steps of:

(1) trifluoromethanesulfonation;

(2) hydride reduction or nucleophilic substitution, and

(3) removal of carboxy and/or amino-protective groups.

One preferred group of Formula II compounds is given in Table II, along with the designations of $R^9$, $R^{10}$, $R^{11}$ and $R^{12}$ for same.

| Table II: Compounds $II_{A-L}$ | | | | |
|---|---|---|---|---|
| Compound | $R^9$ | $R^{10}$ | $R^{11}$ | $R^{12}$ |
| $II_A$ | H | OH | ß-D-Xylose | $OC_2H_5$ |
| $II_B$ | H | OH | ß-D-Xylose | $OCH_2CH_2F$ |
| $II_C$ | H | OH | ß-D-Xylose | $OCH_2CH=CH_2$ |
| $II_D$ | H | OH | ß-D-Xylose | $OCH_2Ph$ |

| Table II: Compounds $II_{A-L}$ (con't) | | | | |
|---|---|---|---|---|
| Compound | $R^9$ | $R^{10}$ | $R^{11}$ | $R^{12}$ |
| $II_E$ | H | OH | ß-D-Xylose | $OCH_2COOH$ |
| $II_F$ | H | OH | ß-D-Xylose | $OCH_2CONH_2$ |
| $II_G$ | $CH_3$ | $NHCH_3$ | ß-D-Xylose | H |
| $II_H$ | $CH_3$ | $NHCH_3$ | H | H |
| $II_I$ | H | OH | ß-D-Xylose | H |
| $II_J$ | H | OH | ß-D-Xylose | $N(CH_3)_2$ |
| $II_K$ | H | OH | ß-D-Xylose | $CONH_2$ |
| $II_L$ | H | OH | ß-D-Xylose | CN |

The synthetic (17-$COOCH_3$) intermediates, compounds $A^1$ through $D^1$, used to produce compounds $II_A$ through $II_L$ are shown in Table III:

| Table III: Compounds $A^1$ through $D^1$ | | | | |
|---|---|---|---|---|
| Compound | $R^9$ | $R^{10}$ | $R^{11}$ | $R^{12}$ |
| $A^1$ | H | OH | ß-D-Xylose | OH |
| $B^1$ | $CH_3$ | $NH_3Cbz$ | ß-D-Xylose | OH |
| $C^1$ | $CH_3$ | $NCH_3Cbz$ | H | OH |
| $D^1$ | H | OH | ß-D-Xylose | OTf |

Cbz = benzyloxycarbonyl

Tf = trifluoromethanesulfonyl

The C-11 modified derivatives of Table II are produced from the 11-hydroxy derivatives above and include

11-O-(substituted)alkylpradimicin T1s, which were prepared by treatment of carboxy protected 11-hydroxypradimicin T1 with an appropriate (and, optionally substituted) alkyl- or aralkylhalide such as ethyl bromide, 2-fluoroethyl bromide, allyl bromide, benzyl bromide, ethyl bromoacetate or iodoacetamide, in an organic solvent, such as dimethylsulfoxide, in the presence of alkali metal salts such as $K_2CO_3$ for 2 to 16 hours, followed by deblocking of the protective group by alkaline hydrolysis and purification by reversed phase column chromatography. The position of newly introduced alkyl groups was established to be at the 11-OH position by the nuclear Overhauser effect observed between 11-O-alkyl-protons and 10- and 12-protons in 'H-NMR.

This group of C-11 modified derivatives include 11-deoxy, 11-dimethylamino, 11-cyano and 11-carbamoyl derivatives of pradimicins A, B and T1, which were prepared from the carboxy and amino protected 11-hydroxypradimicins by sequential reactions as follows:

1) Selective trifluoromethanesulfonylation of 11-hydroxy group by treatment with a triflating reagent such as N-phenyltrifluoromethanesulfonimide or trifluoromethanesulfonic anhydride in a basic organic solvent such as pyridine in the presence of an organic base such as dimethylaminopyridine at 0°C to room temperature for 30 minutes to 16 hours, followed by chromatographic purification on a reversed phase column afforded the corresponding 11-O-trifluoromethanesulfonyl intermediates in 58-85% yield. Their structures were confirmed by the characteristic low field shifts of 10- and 12-protons in $^1$H-NMR;

2) and reactions of the 11-O-triflates in an organic solvent such as DMF with hydride ion generated by the combined addition of formic acid and triethylamine, with nucleophilic base such as dimethylamine or with trialkyl substituted metal reagent such as tributyltin cyanide in the presence of palladium (O) catalyst, such as tetrakis(triphenylphosphine)palladium(O), or generated in situ from palladium acetate and triphenylphosphine for the reaction period of 30 minutes to 16 hours at room temperature or under heating at 50-100°C, followed by deblocking of the carboxy and amino protective group(s) by alkaline hydrolysis, with optimal hydrogenation and purification by $C_{18}$ reversed phase column chromatography yielded the corresponding 11-modified derivatives.

*In Vitro* Antifungal Activity

The *in vitro* antifungal activity (MIC) of the compounds of the invention was determined on yeast morphology agar (YMA, Difco Laboratories, USA), buffered with 0.06M phosphate (pH 7.0). Nine parts of molten agar were combined with one part of antibiotic dilution in petri dishes. A 5-µl suspension containing $2x10^6$ cells/ml was spotted on the surface of the agar plates. The plates were incubated at 28°C and MICs were recorded after 40 hours of the incubation. The results are summarized in Table 1. All of the 11-hydroxy derivatives of Formula I-I are active against a wide range of fungi. The C-11 modified derivatives of Formula II showed various ranges of antifungal activity.

Table IV: In vitro Antifungal Activity of C-11 Modified Derivatives of Pradimicins

| Compound | MIC (μg/ml) Ca-4 | Ct-12 | Cn-2 |
|---|---|---|---|
| **Pradimicin A** | **6.3** | **12.5** | **1.6** |
| $I_A$ | 6.3 | 25 | 3.1 |
| $I_C$ | 6.3 | 12.5 | 1.6 |
| $I_D$ | 6.3 | 12.5 | 3.1 |
| $I_E$ | 6.3 | 25 | 6.3 |
| $I_F$ | 6.3 | 12.5 | 12.5 |
| $II_A$ | 3.1 | 12.5 | 1.6 |
| $II_B$ | 3.1 | 6.3 | 1.6 |
| $II_C$ | 6.3 | 12.5 | 3.1 |
| $II_D$ | >100 | >100 | 12.5 |
| $II_G$ | 6.3 | 12.5 | 1.6 |

Table IV: (con't.) In vitro Antifungal Activity of C-11 Modified Derivatives of Pradimicins

| | MIC (μg/ml) | | |
|---|---|---|---|
| $II_I$ | 6.3 | 12.5 | 6.3 |
| $II_J$ | 12.5 | >100 | 50 |
| $II_K$ | 12.5 | 25 | 6.3 |
| $II_L$ | 12.5 | 25 | 6.3 |

Ca-4: Candida albicans A9540
Ct-12: Candida tropicalis IFO10241
Cn-2: Cryptococcus neoformans IAM4514

11-xylosyl Precursors

This section describes the preparation of the 11-xylosyl compounds from which the compounds of the invention are derived. The compounds, characteristics and their biological properties are discussed.

A. The Compounds

The antibiotic substances, named pradimicins T1 and T2, 11-*O*-dexylosypradimicin T1, were prepared via the fermentation of Actinomycete strain AA3798 (ATCC Deposit No. 55288). The use of these compounds in the treatment of infectious diseases caused by microorganisms susceptible to said antibiotics is discussed later.

The features of structure B are common to all three of the compounds. Variations of $R^d$ and $R^e$, as indicated below, yield individual structures.

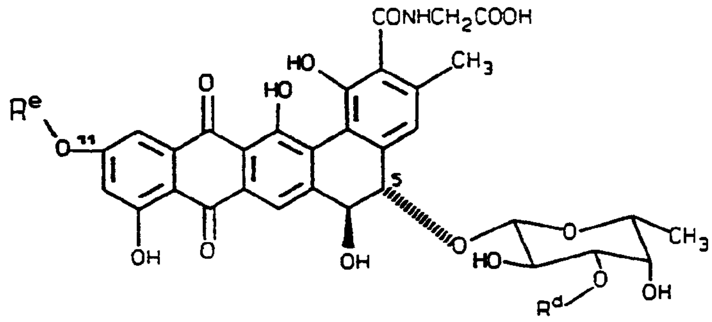

(B)

| | $R^d$ | $R^e$ |
|---|---|---|
| Pradimicin T1: | 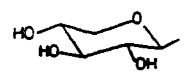 | 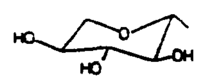 |
| Pradimicin T2: | H | 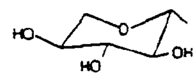 |
| 11-O-Dexylosylpradimicin T1: | 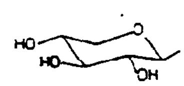 | H |

As part of an effort to screen for water-soluble analogs of pradimicin, applicants discovered that a rare actinomycete numbered AA3798 produced new members of the pradimicin family of antibiotics. Strain AA 3798 was isolated from a sample of soil picked up at Nerima, Tokyo, Japan on January 29, 1989.

A culture of strain AA 3798 was deposited with the American Type Culture Collection under the BUDAPEST TREATY ON THE INTERNATIONAL RECOGNITION OF THE DEPOSIT OF MICROORGANISMS FOR THE PURPOSES OF PATENT PROCEDURE, and all restrictions on the availability to the public of deposited microorganism will be irrevocably removed upon the granting of a patent from this application. The deposited culture has been assigned the accession numbered ATCC 55288.

Taxonomy of Strain AA 3798

The media used in the study on the cultural and physiological characteristics of the strain were prepared according to the recommendation by Shirling and Gottlieb in "Methods for Characterization of *Streptomyces* Species," Intern. J. Syst. Bact., 1966, 16:313-340; by Waksman in The Actinomycetes, Vol. II, "Classification, Identification and Description of Genera and Species", pp. 328-334, published by The Williams and Wilkins Company, Baltimore, 1961; and by Arai in Culture Media for Actinomycetes, published by The Society for Actinomycetes Japan, 1975. Cultures were observed after incubation at 32°C for two to four weeks. Color names and hue numbers (Table 1) are given according to the Manual of Color Names (Japan Color Enterprise Company, Ltd., 1987).

Morphology

Strain AA 3798 grew well on yeast extract-malt extract agar (ISP-2), inorganic salt-starch agar (ISP-4), yeast starch agar and Bennett's agar at any temperature between 19 and 40°C. Colonies were thickly covered with a powdery to velvety aerial mycelium. The substrate mycelium was well developed and irregularly branched. On some agar media such as water agar, this substrate mycelium had fragmented, irregularly zig-zagged, into short rod elements. The aerial mycelia were abundant and long, moderately branched, straight or irregularly curved. Aerial mycelia were so fine (0.3 μm in diameter) that their sporulation could not be observed under a light microscope, but scanning electron microscopy revealed that they were completely fragmented into spores. The spores were bacillar-shaped and measured 0.3 x 1.0 - 2.0 μm in size, and with a smooth surface. These spores were not motile.

B. Characteristics

Strain AA 3798 had white aerial mycelia; upon prolonged incubation, the aerial mycelia sometimes turn pinkish white. The color of vegetative and diffusible pigments on organic media ranged from soft pink to dark red. The color changed from pale reddish yellow to strong yellowish orange by the addition of 0.1N HCl. The cultural characteristics of strain AA 3798 on various media are summarized in Table V.

Table V. Cultural characteristics of strain AA 3798

| Medium | | |
|---|---|---|
| Sucrose nitrate agar (Waksman med. No. 1) | Growth | Pale reddish yellow (130) |
| | Reverse | Soft yellowish pink (27) |
| | Aerial mycelium | White (388) powdery |
| | Soluble pigment | Soft yellowish pink (28) |
| Glycerol nitrate agar | Growth | Pale reddish yellow (126) |
| | Reverse | Light reddish yellow (131) |
| | Aerial mycelium | White (388) powdery |
| | Soluble pigment | None |
| Glucose asparagine agar (Waksman med. No. 2) | Growth | Light orange (64) |
| | Reverse | Light orange (64) |
| | Aerial mycelium | White (388) powdery |
| | Soluble pigment | None |
| Yeast extract-malt extract agar (ISP med. No. 2) | Growth | Dark Red (57) |
| | Reverse | Dark red (57) |
| | Aerial mycelium | Pinkish white (391) powdery, good |
| | Soluble pigment | Dark red (57) |
| Oatmeal agar (ISP med. No. 3) | Growth | Colorless-grayish pink (33) |
| | Reverse | Soft yellowish pink (27) |
| | Aerial mycelium | White (388) powdery |
| | Soluble pigment | Soft pink (25) |
| Inorganic salts-starch agar (ISP med. No. 4) | Growth | Yellowish brown (101) |
| | Reverse | Soft orange (83) |
| | Aerial mycelium | White (388) powdery, good |
| | Soluble pigment | None-beige white (392) |
| Glycerol asparagine agar (ISP med. No. 5) | Growth | Light orange (64) |
| | Reverse | Light orange (65) |
| | Aerial mycelium | White (388) powdery |
| | Soluble pigment | None |

| Table V. (continued) Cultural characteristics of strain AA 3798 | | |
|---|---|---|
| Medium | | |
| Tyrosine agar (ISP med. No. 7) | Growth | Light orange (65) |
| | Reverse | Light orange (65) |
| | Aerial mycelium | Beige white (392) powdery |
| | Soluble pigment | None |
| Nutrient agar (Waksman med. No. 14) | Growth | Pale reddish yellow (125) |
| | Reverse | Pale reddish yellow (125) |
| | Aerial mycelium | None |
| | Soluble pigment | None |
| Yeast starch agar | Growth | Dark red (57) |
| | Reverse | Dark red (57) |
| | Aerial mycelium | White (388) powdery, good |
| | Soluble pigment | Strong purplish red (44) |
| Bennett's agar (Waksman med. No. 30) | Growth | Dark red (57) |
| | Reverse | Dark red (57) |
| | Aerial mycelium | White (389) powdery, good |
| | Soluble pigment | Strong purplish red (44) |
| Gauze's agar | Growth | Pale reddish yellow (125) |
| | Reverse | Light orange (64) |
| | Aerial mycelium | White (388) powdery, good |
| | Soluble pigment | Pinkish white (391) |
| Peptone corn agar | Growth | Grayish pink (32) |
| | Reverse | Grayish pink (32) |
| | Aerial mycelium | White (389) powdery, scant |
| | Soluble pigment | Soft pink (25) |

Physiological characteristics

The physiological characteristics and utilization of carbon sources of strain AA 3798 are shown in Tables VI and VII, respectively. No vitamins were essential for growth.

| Table VI. Physiological characteristics of strain AA 3798 | |
|---|---|
| Test | Results |
| Starch hydrolysis (on ISP med. No. 4) | Positive |
| Nitrate reduction (Difco, nitrate broth) | Negative |
| Milk (Difco, 10% skimmed milk)<br>Coagulation<br>Peptonization | <br>Positive<br>Positive |
| Cellulose decomposition (sucrose nitrate solution with a paper strip as the sole carbon source) | Negative |
| Gelatin liquefaction<br>On plain gelatin<br>On glucose peptone gelatin | <br>No growth<br>No growth |
| Melanin formation (On ISP med. No. 7) | Negative |
| Temperature range for growth (°C)<br>Optimum temperature (°C)<br>(On Yeast starch agar) | 19 - 40<br>29 - 34 |
| pH range for growth<br>Optimum pH<br>(On Trypticase soy broth, BBL) | 6 - 8<br>7 - 8 |
| Growth in/at<br>Lysozyme 0.01%<br>0.1%<br>NaCl 2%<br>8% | <br>Positive<br>Negative<br>Positive<br>Negative |

| Table VII. Utilization of carbon sources by strain AA 3798 | |
|---|---|
| Carbon Source | Growth |
| Arabinose | ++ |
| Inositol | ++ |
| D-Mannitol | ++ |
| Sucrose | ++ |
| L-Rhamnose | ++ |
| Raffinose | ++ |
| D-Xylose | ++ |
| D-Glucose | ++ |
| Fructose | + |

+: moderate growth, ++: abundant growth

Antibiotic susceptibility of strain AA 3798 was examined using antibiotic disks (Tridisk, Eiken Chemical Co., Ltd). The disks were placed onto the surface of yeast-glucose-malt agar (yeast extract 0.1%, glucose 1%, malt extract 0.1%, $CaCl_2 \cdot 2H_2O$ 0.05%, and agar 1.5%) which had been seeded by strain AA 3798 (4% inoculum), and the plates were then incubated at 37°C for 4 days.

Strain AA 3798 was resistance to 20 μg of ampicillin, 10 μg of cephalexin, 50 μg of fosfomycin, 15 μg of lincomycin, 5 μg of gentamicin, 10 μg of tobramycin, 15 μg of nalidixic acid, 300 U of polymyxin B, 10 μg of erythromycin and 15 μg of josamycin. It was susceptible to 1 μg of clavulanic acid, 2 μg of ticarcillin, 30 μg of tetracycline, 10 μg of chloramphenicol, 30 μg of kanamycin, 2 μg of norfloxacin and 50 U of colistin.

Cell wall chemotype

Whole-cell compositions were analyzed by the procedures described by Becker and Lechevalier in "Rapid Differentiation between *Nocardia* and *Streptomyces* by Paper Chromatography of Whole Cell Hydrolysate," Appl. Microbiol., 1964, 12:421-423, and in "Chemical Compositions of Cell-Wall Preparations from Strains of Various Form-Genera of Aerobic Actinomycetes," Appl. Microbiol., 1965, 13:236-243. Strain AA 3798 contained *meso*-diaminopimeric acid, glucose, glucosamine, galactose and small amounts of ribose, mannose and xylose, but madurose and arabinose were not detected, indicating that the cell wall of this strain is type IIIC. Mycolic acids were not detected by the method of Minnikin *et al* in "Differentiation of *Mycobacterium, Nocardia,* and Related Taxa by Thin-Layer Chromatographic Analysis of Whole-Organism Methanolysates," J. Gen. Microbiol., 1975, 88:200-204. Analysis of the menaquinone composition using the procedure of Collins *et al* in "A Note on the Separation of Natural Mixtures of Bacterial Menaquinones Using Reverse-Phase Thin-Layer Chromatography," J.Appl. Bacteriol., 1980, 48:277-282, revealed 69% MK-9($H_8$), 18% MK-9($H_6$), 6% MK-9($H_4$) and 6% MK-9($H_{10}$). The whole cell fatty acids determined by the method of Suzuki *et al* in "Taxonomic Significance of Cellular Fatty Acid Composition in Some Coryneform Bacteria," Int. J. Syst. Bacteriol., 1983, 33:188-200, consisted of 34% 14-methylpentadecanoic acid (*iso*16:0), 21% 14-methylhexadecanoic acid (*anteiso*-17:0), 14% 15-methylhexadecanoic acid (*iso*17:0) and 10% 10-methyloctadecanoic acid (10Me18:0).

According to the guide to generic identification of Actinomycetes described by H.A. Lechevalier (in Bergey's Manual of Systematic Bacteriology, Vol. 4., Eds. S.T. Williams et al, pp. 2344-2347, 1989, Williams & Wilkins), the presence of *meso*-diaminopimelic acids together with no diagnostic sugar and long chains of spores formed on the aerial hyphae indicate that strain AA 3798 belongs to the genus *Nocardiosis* as only one generic possibility. However, on the basis of the definition described by Grund and Kroppenstedt ("Fermentation, Chemotaxonomy and Numerical Taxonomy of the Genus *Nocardiosis* Meyer", 1976, Int. J. Syst. Bacteriol. 40:5-11, 1990), the major components of both menaquinones and fatty acids from strain AA 3798 are quite different from those of genus *Nocardiosis.* Since no actinomycetous organisms with the cell wall chemotype as described above have been reported previously, it is impossible to assign the genus of strain AA 3798 at present, although the proposal of a new genus might be justified. Thus, strain AA 3798 was assigned as an unidentified actinomycete.

The novel antibiotics of this invention are produced during the aerobic fermentation of suitable liquid nutrient media under controlled conditions via inoculation with the above strain or its variants or mutants. Liquid nutrient media such as those employed for the production of common antibiotics are suitable for producing pradimicins T1 and T2. Desirable carbon sources are glucose, glycerol, fructose, sucrose, xylose, starch and other carbohydrates and preferable nitrogen sources are soybean meal, cotton seed meal, peptone, meat extract, fish meals, corn steep liquor and the like.

Desirable inorganic salts are cobalt chloride, calcium carbonate and potassium phosphate, which are added if necessary. A preferable liquid medium is the one described in Example 2. Another more convenient medium is FR-22, which is composed of soybean meal (1-3%), glucose (1-3%), D-aspartic acid (0.1-0.2%) and $CaCO_3$(0.05-0.3%). Adekanol, silicone and the like are used as the antifoaming agents. As every microbiologist who is experienced in fermentation will appreciate, the production and ratio of pradimicins T1 and T2 vary according to the fermentation conditions and the strains and/or media used. Preferred fermentation conditions are aerobic cultivations at pH 5-8 at 20-37°C for 5-14 days, preferably at pH 6-7 at 28-34°C for 6-12 days.

Production of pradimicins T1 and T2 can be readily monitored during the course of the fermentation by conventional techniques such as thin layer chromatography, HPLC, visible absorption and anti-*Candida* activity.

## Isolation and purification

For the isolation of pradimicins T1 and T2 from the fermentation broth, separation methods which are usually used to isolate metabolites produced by microorganisms from their cultured broth can properly be used. As pradimicins T1 and T2 are water-soluble acidic substances and predominantly produced in the fermentation broth, they are recovered advantageously by usual procedures for hydrophilic acidic substances such as organic solvent extraction, ion exchange resin, acidic precipitation, partition chromatography and the like; either alone or in combination. For example, after completion of the fermentation, the whole fermentation broth is centrifuged or filtered. The resulting supernatant or filtrate is adsorbed on high porous polymer resin such as Diaion HP-20 (Mitsubishi Kasei Co.) and eluted with water miscible organic solvent such as methanol acetonitrile or acetone. The eluate is concentrated *in vacuo.* Precipitation in acetone or lyophilization gave crude pradimicins T1 and T2. For purification, the crude material is applied onto a reverse phase silica gel column such as YMC-ODS A60 (Yamamura Chemical Lab.) and eluted with acetonitrile:0.15% phosphate buffer, pH 3.5, (14.5:85.5, V/V), yielding pure pradimicins T1 and T2.

Pradimicin T1 may be converted to its 11-*O*-dexylosyl derivative. Heating pradimicin T1 in an alkaline medium for a period sufficient to cleave the xylosyl group at the 11-*O*-position provides the corresponding 11-*O*-dexylosyl derivative. The solvent used may be for example dioxane, tetrahydrofuran, water, lower alkanol, or mixtures thereof; alkaline hydroxide may be for example sodium hydroxide, potassium carbonate, lithium hydroxide. The temperature may be from about 60°C to about 100°C, or the reflux temperature of the solvent.

Reaction time may be from about 1 to 10 hrs and will depend on the reaction conditions employed.

Seed culture

An actinomycete strain AA 3798 was propagated on YSM agar slant composed of soluble starch 1%, yeast extract (Difco laboratories) 0.1%, soytone (Difco laboratories) 0.1%, $CaCl_2 \cdot 2H_2O$ 0.05% and agar 1.6%, at 32°C for 20 days. A portion of the mature agar slant was inoculated into a 500-ml Erlenmeyer flask containing 100 ml of the medium V15-1 composed of glucose 1.5%, peptone (Daigo Eiyo Co.) 0.2%, NZ-case (Scheffield Products) 0.2%, meat extract (Mikuni Kogyo Co.) 1.0%, $NaNO_3$ 0.2%, $K_2HPO_4$ 0.1%, $MgSO_4 \cdot 7H_2O$ 0.05%, and $CaCl_2 \cdot 2H_2O$ 0.05% and then incubated at 32°C for five days on a rotary shaker with orbiting at 200 rpm. The resulting vegatative mycelia were washed and resuspended with a half volume of 20% aq. glycerol and then stored at -80°C. The seed culture for flask fermentation was prepared by inoculating 1 ml of frozen vegetative mycelia in 100 ml of the medium V15-1 in a 500-ml Erlenmeyer flask and shaking for four days at 32°C.

Flask fermentation

Each 5-ml portion of the seed culture as set forth in the aforesaid example of seed culture was transferred into 500-ml Erlenmeyer flasks containing 100 ml of production medium 84B composed of soybean meal (Nikko Seiyu Co.) 3%, Pharmamedia (Trader's Protein) 0.5%, glucose 3%, yeast extract (Oriental Yeast Co.) 0.1% and $CaCO_3$ 0.3%. The pH was adjusted to 7.0 before autoclaving. The fermentation was conducted on a rotary shaker operating at 200 rpm for 12 days at 28°C. The production of the antibiotics was monitored by measuring the visible absorption at 500 nm in 0.02N NaOH:MeOH (1:1) solution. For quantitative analysis of pradimicins T1 and T2, the supernatant was diluted ten fold with dimethylsulfoxide, and filtered (Ekicrodisc 13CR, Pore size: 0.45 μm, Gelman Science Japan, Ltd.), and the filtrate (10 μl) was analyzed by HPLC on Excel pak SIL-C185R (Yokogawa Electronic Co., Ltd.) using acetonitrile: 0.15% phosphate buffer, pH 3.5 (19:81) at a flow rate of 1 ml/min with 460 nm detection. The total titer of pradimicins T1 and T2 reached about 900 μg/ml after 12 days of fermentation (pradimicin T1: 680 μg/ml, pradimicin T2: 230 μg/ml).

Alternative flask fermentation

Each 5-ml portion of the seed culture as set forth in the aforesaid example of seed culture was transferred into 500-ml Erlenmeyer flasks containing 100 ml of the production medium, named FR-22, which was composed of defatted soybean meal 3%, glucose 3%, D-aspartic acid 0.2% and $CaCO_3$ 0.3% (pH was adjusted to 7.0 before autoclaving). The fermentation was carried out on a rotary shaker at 28°C for eight days, during which the production of the antibiotic reached about 1,400 μg/ml (pradimicin T1: 820 μg/ml, pradimicin T2: 520 μg/ml).

Isolation of pradimicins T1 and T2

The fermentation broth as set forth under "Flask fermentation" was centrifuged at 3,000 rpm for 20 min. The supernatant (3.5 L) was adsorbed on a column (2.2 L) of Diaion HP-20. The resin was eluted with 40% aq. methanol (5.3 L) after washing with water (7 L) and 20% aq. methanol (5 L). The eluate was concentrated *in vacuo* and lyophilized to yield a crude solid (5.9 g). This solid (4.9 g) was dissolved in water (2 L), the pH was adjusted to 2.0 with 1N HCl, and the product was extracted with *n*-butanol (2 L). The organic layer was back-extracted with alkaline water (pH 8.5, 0.6 L) and the aqueous layer was concentrated after adjusting to pH 7.0. The aqueous residue (0.2 L) was dropwise added to acetone (1.8 L) and the resulting dark red powder (2.5 g) was collected by filtration. This powder (2.4 g) was dissolved in a mixture of acetonitrile: 0.15% phosphate buffer, pH 3.5, (14.5:85.5, 240 ml) and then subjected to chromatography on a column of YMC-gel, ODS-A60 (10 L) which had been equilibrated with the same solvent. The eluates with the same solvent were monitored by HPLC (column: YMC A30, 4.5 mm ID x 100 mm, 3 μm, Yamamura Chemical Lab., mobile phase: acetonitrile: 0.15% phosphate buffer, pH 3.5, (19:81)). The fractions either. containing pradimicin T1 or pradimicin T2 were pooled, concentrated and then desalted to afford pure pradimicin T1 (293 mg) and pradimicin T2 (79 mg).

Preparation of 11-*O*-Dexylosylpradimicin T1

A solution of pradimicin T1 (500 mg) in 80 ml of 1N sodium hydroxide was heated at 65°C for seven hours. The reaction mixture was cooled, adjusted to pH 3.0 with 6N HCl, and the solid formed was collected by centrifugation (3,000 rpm). The solid was charged on a column of YMC gel ODS-A60 (1,350 ml) and eluted with $CH_3CN$: 0.15% phosphate buffer, pH 7.0 (7:93, 15L). The fractions containing 11-*O*-dexylosylpradimicin T1 were pooled, concentrated *in vacuo* and passed through a short column of Diaion HP-20. The column was washed with water and eluted with 60% aqueous acetone (500 ml). Concentration of the eluate *in vacuo* and lyophilization from water gave a dark red powder (294 mg, 68%).

Physico-chemical properties

Physico-chemical properties of pradimicins T1 and T2, and 11-*O*-dexylosylpradimicin T1 are summarized in Table 4.

Table VIII. Physico-chemical properties of pradimicins T1 and T2, 11-O-dexylosylpradimicin T1

| | Pradimicin T1 | Pradimicin T2 | 11-O-dexylosyl-Pradimicin T1 |
|---|---|---|---|
| Appearance: | Dark red amorphous | Dark red amorphous | Dark red amorphous |
| M.P. (dec.): | 190-195°C | 185-190°C | 200-210°C |
| $[\alpha]_D^{30}$ (c 0.05, 0.1N HCl): | Unable to determine | Unable to determine | Unable to determine |
| FAB-MS (m/z): | 932(M + H)$^+$ | 800(M + H)$^+$ | 800(M + H)$^+$ |
| Molecular formula: | $C_{42}H_{45}NO_{23}$ | $C_{37}H_{37}NO_{19}$ | $C_{37}H_{37}NO_{19}$ |
| UV $\lambda_{max}$ nm (ε) | | | |
| in 0.02N NaOH-MeOH(1:1): | 243(33,600)<br>316(14,000)<br>500(14,200) | 242(29,500)<br>316(12,200)<br>499(12,200) | 246(32,500)<br>308(21,300)<br>505(15,300) |
| in 0.02N HCl-MeOH(1:1): | 232(33,100)<br>290(25,300)<br>457(11,000) | 233(29,600)<br>290(22,600)<br>456(9,800) | 232(28,500)<br>299(23,400)<br>459(8,800) |
| in MeOH-$H_2O$(1:1): | 224(30,400)<br>274(25,100)<br>493(10,100) | 220(27,800)<br>274(23,500)<br>501(9,800) | 230(31,800)<br>288(23,800)<br>477(9,500) |
| Solubility in solvent: | | | |
| Soluble: | Dimethyl sulfoxide, N,N-dimethylformamide and alkaline water, etc. | | |

| Table VIII. (con't) Physico-chemical properties of pradimicins T1 and T2, 11-O-dexylosylpradimicin T1 | | | |
|---|---|---|---|
| Slightly soluble: | Ethanol, methanol and acetone, etc. | | |
| Insoluble: | Benzene, chloroform, etc. | | |
| TLC ($SiO_2$), Rf: | | | |
| n-BuOH-AcOH-$H_2O$=2:1:1 | 0.48 | 0.50 | 0.70 |
| n-BuOH-AcOH-Pyridine-$H_2O$=6:1:4:3 | 0.28 | 0.38 | 0.57 |
| HPLC Rt (min) (ODS, $CH_3CN$-0.15% $KH_2PO_4$, pH 7.0 = 12:88 v/v) | 7.5 | 4.1 | 11.0 |

Biological properties

The *in vitro* antifungal activities of pradimicins T1 and T2 and 11-*O*-dexylosylpradimicin T1 were determined by the agar dilution method on yeast morphology agar containing 1/15M phosphate buffer (pH 7.0). The MICs for individual isolates are shown in Table IX.

| Table IX. In vitro antifungal activity | | |
|---|---|---|
| Test organism | MIC (μg/ml)* | |
| Saccharomyces cerevisiae ATCC 9763 | Pradimicin T1 | 3.1 |
| | Pradimicin T2 | 0.8 |
| | 11-O-Dexylosyl-pradimicin T1 | 3.1 |
| | Amphotericin B | 0.4 |
| | Ketoconazole | 50 |
| Candida albicans A9540 | Pradimicin T1 | 6.3 |
| | Pradimicin T2 | 3.1 |
| | 11-O-Dexylosyl-pradimicin T1 | 6.3 |
| | Amphotericin B | 0.4 |
| | Ketoconazole | 50 |

| Table IX. (continued) in vitro antifungal activity | | |
|---|---|---|
| Test organism | Compound | MIC(μg/ml) |
| Candida albicans ATCC 38247 | Pradimicin T1<br>Pradimicin T2 | 1.6<br>1.6 |
| | 11-O-Dexylosyl-pradimicin T1 | 3.1 |
| | Amphotericin B | 3.1 |
| | Ketoconazole | 25 |
| Candida albicans ATCC 32354 (B311) | Pradimicin T1<br>Pradimicin T2 | 6.3<br>3.1 |
| | 11-O-Dexylosyl-pradimicin T1 | 3.1 |
| | Amphotericin B | 0.4 |
| | Ketoconazole | 50 |
| Candida albicans 83-2-14 (Juntendo) | Pradimicin T1<br>Pradimicin T2 | 6.3<br>3.1 |
| | 11-O-Dexylosyl-pradimicin T1 | 3.1 |
| | Amphotericin B | 0.4 |
| | Ketoconazole | 12.5 |
| Candida tropicalis 85-8 (Kitasato) | Pradimicin T1<br>Pradimicin T2 | 25<br>6.3 |
| | 11-O-Dexylosyl-pradimicin T1 | 12.5 |
| | Amphotericin B | 0.8 |
| | Ketoconazole | 100 |
| Candida tropicalis IFO 10241 | Pradimicin T1<br>Pradimicin T2 | 12.5<br>6.3 |
| | 11-O-Dexylosyl-pradimicin T1 | 12.5 |
| | Amphotericin B | 0.8 |
| | Ketoconazole | 50 |
| Cryptococcus neoformans D49 | Pradimicin T1<br>Pradimicin T2 | 3.1<br>6.3 |
| | 11-O-Dexylosyl-pradimicin T1 | 6.3 |
| | Amphotericin B | 0.4 |
| | Ketoconazole | 0.8 |

| Table IX. (continued) in vitro antifungal activity | | |
|---|---|---|
| Test organism | Compound | MIC(µg/ml) |
| Cryptococcus neoformans IAM 4514 | Pradimicin T1<br>Pradimicin T2 | 3.1<br>6.3 |
| | 11-O-Dexylosyl-pradimicin T1 | 6.3 |
| | Amphotericin B | 0.4 |
| | Ketoconazole | 0.4 |
| Aspergillus fumigatus IAM 2034 | Pradimicin T1<br>Pradimicin T2 | 6.3<br>>100 |
| | 11-O-Dexylosyl-pradimicin T1 | 25 |
| | Amphotericin B | 0.8 |
| | Ketoconazole | 3.1 |
| Trichophyton mentagrophytes 4329 | Pradimicin T1<br>Pradimicin T2 | 6.3<br>50 |
| | 11-O-Dexylosyl-pradimicin T1 | 12.5 |
| | Amphotericin B | 0.8 |
| | Ketoconazole | 0.8 |

**Medium:** Yeast morphology agar + 1/15M phosphate buffer (pH 7.0)
**Inoculum:** $10^6$ cells/ml (except *T. mentagrophytes*: $10^7$ cells/ml)
**Incubation conditions:** 28°C, 40 hrs. (60 hrs. *T. mentagrophytes*)

Pradimicins T1 and T2 were evaluated for *in vivo* efficacy against a *Candida albicans* A9540 systemic infection in male ICR mice (20-24 g body weight) as described by Oki *et al.* in "Water-soluble pradimicin derivatives, synthesis and antifungal evaluation of N,N,-dimethyl pradimicins", J. Antibiotics 43:1230-1235, 1990. Five mice at each dose level were challenged intravenously with ten times the 50% lethal dose of the pathogen suspended in saline. Antibiotics were intravenously administered once immediately after the fungal challenge. Infection control animals (n=10) died from 7 to 12 days. The 50% protective dose ($PD_{50}$) was calculated from the survival rate 20 days after the fungal infection. The acute toxicity of pradimicins T1 and T2 was determined in mice after a single intravenous dose. The $PD_{50}$ and $LD_{50}$ of pradimicins T1 and T2 are presented in Table X.

| Table X. *In vivo* activity against *C. albicans* A9540 systemic infection in mice | | |
|---|---|---|
| Compound | $PD_{50}$ (mg/kg) | $LD_{50}$ (mg/kg) |
| Pradimicin T1<br>Pradimicin T2 | 27<br>>50 | 300<br>>600 |
| Amphotericin B | 0.2 | 4.5 |
| Ketoconazole | >50 | NT |

NT: Not tested

## Compositions

Compositions employing one or more of the compounds of the invention and/or their acid or base addition salts may contain suitable amounts of pharmaceutically acceptable excipients.

By "acid or base addition salts", applicants mean compounds or complexes formed when the subject compounds are contacted with acidic or basic reagents conventionally employed to yield bioavailable forms of therapeutic agents. Suitable reagents include hydrochloric and, oxalic acid, sodium hydroxide, potassium hydroxide, lithium hydroxide, sodium carbonate and the like.

The compounds of the invention will generally be present in formulations with about 0.001 to about 99.99% of one or more excipients in formulations to be administered via oral, buccal, nasal, topical or parenteral dosage forms. Parenteral dosage forms are preferred. Useful excipients include carriers, colorants, perfumes, flow control agents, stabilizers and the like. The content of drug in formulations usually 50 to about 90 wt% useful amounts of the therapeutic agents range from about 5 mg/kg to about 100 mg/kg of patient body weight or host weight. Suitable "patients" or "hosts" include mammals preferable humans, and a variety of other organisms.

## EXAMPLES

The following examples serve to illustrate the invention.

### Example 1: 11-*O*-Demethylpradimicin A($I_A$) and 11-*O*-Demethylpradimicin B($I_B$)

A mixture of pradimicin A (420mg, 0.5 mmol) and anhydrous lithium iodide (1.34g, 10 mmol) in collidine (50 ml) was refluxed for 2 hours with stirring and then cooled. The mixture was diluted with ether (200ml) and filtered. The solid was dissolved in water (100ml) and the solution was acidified with 1N-HCl and charged on an HP-20 (1000 ml) column, which was washed with pH 3.5 phosphate buffer and then eluted with aqueous 50% acetonitrile. The fractions containing the desired products were combined and concentrated to remove most of acetonitrile. The concentrate was charged on a reversed phase column (Prep $C_{18}$. 55-105 μm, Waters, 400 ml), which was washed with 10% acetonitrile in pH 3.5 phosphate buffer and then eluted with 25 and 30% acetonitrile in pH 3.5 phosphate buffer. The crude fractions containing a mixture of $I_A$ and $I_B$ were desalted by $C_{18}$ column chromatography and freeze-dried to give 100 mg of a mixture, which was purified by a preparative-HPLC column (Nihon seimitsu NC-20250, ODS 5μ, 20 x 250 mm) eluting with 27.5% acetonitrile in pH 3.5 phosphate buffer. The first fractions showing retention time 4.1 min. [25% acetonitrile-buffer (pH 3.5)] were combined, concentrated, desalted and freeze-dried to give 29 mg of 11-*O*-demethylpradimicin A ($I_A$). The second fractions gave 40 mg of a mixture of $I_A$ and $I_B$. The third fractions (retention time, 4.5 min.) were purified by a similar procedure to give 12 mg of 11-*O*-demethylpradimicin B ($I_B$). The second fractions (40 mg) was repurified by preparative-HPLC using the same column and solvent to give an additional 10 mg of $I_A$ and 5 mg of $I_B$. The total yield of $I_A$ and $I_B$ was 39 mg (9.4%) and 17 mg (5%), respectively.

Compound $I_A$: Mp >200°C;IR $\nu_{max}$ (KBr) $cm^{-1}$ 1720, 1610; UV $\lambda_{max}$ (0.01N-NaOH)nm(ε) 502(15500); $^1H$ NMR(400 MHz, DMSO-$d_6$) δ 1.27(3H,d,J=6.8 Hz, 5'-Me), 1.34(3H,d, J=7.3 Hz, 17-Me), 2.30(3H,s, 3-Me), 2.65(3H, s, N-Me), 3.12(1H, t,J=11.1 Hz, 5"-$H_{ax}$), 3.14(1H,dd, J=8.1 & 6.8 Hz, 2"-H), 3.16(1H,dd, J=9.0 & 8.1 Hz, 3"-H), 3.32(1H, m, 4"-H), 3.43(1H, m, 4'-H), 3.75(1H, dd, J=11.1 & 5.1 Hz, 5"-$H_{eq}$), 3.90(1H,q, J=6.8 Hz, 5'-H), 3.95(1H,dd, J=9.4 & 5.1 Hz, 3'-H), 4.40(1H, q, J=7.3 Hz, 17-H), 4.46(1H, d, J=6.8 Hz, 1"-H), 4.53(2H, s, 5 & 6-H), 4.79(1H, d, J=8.1 Hz, 1'-H), 6.55(1H, br s, 10-H), 6.95(1H, s, 4-H), 7.14(1H, br s, 12-H), 7.79(1H, s, 7-H); FAB(+)- MS m/z 827 (M+H).

Compound $I_B$: Mp >200°C; IR $\nu_{max}$ (KBr) $cm^{-1}$ 1720, 1600; UV $\lambda_{max}$ (0.01N-NaOH) nm(ε) 501(11500); $^1H$ NMR(400 MHz, DMSO-$d_6$) δ 1.27(3H, d, J=6.8 Hz, 5'-Me), 1.343H, d, J=7.3 Hz, 17-Me), 2.32(3H, s, 3-Me), 2.70(3H s N-Me), 3.75(1H, m, 3'-H), 3.88(1H, q, J=6.8 Hz, 5'-H), 4.40(1H, q, J=7.3 Hz, 17-H), 4.55(2H, m, 5 & 6-H),4.70(1H,d, J=6.4 Hz, 1'-H), 6.68(1H, d, J=2.6 Hz, 10-H), 7.14(1H, br s, 4-H), 7.25(1H, d, J=2.6 Hz, 12-H), 8.03(1H, br s, 7-H); FAB(+)-MS m/z 695(M+H), 717(M+Na).

### Example 2: 11-O-Demethylpradimicin C($I_C$)

A mixture of pradimicin C (168mg, 0.2 mmol), anhydrous lithium iodide (1.34 g, 10 mmol) and molecular sieves 4A (168 mg) in collidine (20 ml) was refluxed for 4 hours with stirring and cooled. The mixture was diluted with ether (50 ml) and filtered. The solid was dissolved in water (50 ml) and the solution was acidified with N-HCl and charged on an HP-20 (200 ml) column, which was washed with pH 3.5 phosphate buffer and then eluted with aqueous 50% acetonitrile. The fractions containing the desired product were combined and concentrated to remove most of acetonitrile. The concentrate was charged on a reversed phase column (Prep $C_{18}$ 55-105 μm, Waters, 80 ml), which was washed with 10% acetonitrile in pH 3.5 phosphate buffer and then eluted with 25% acetonitrile in pH 3.5 phosphate buffer. The desired fractions were desalted by $C_{18}$ column chromatography and freeze-dried to give 50 mg of the crude product, which was purified by preparative HPLC

column (Nihon seimitsu NC-20250, ODS 5μ, 20 x 250 mm) eluting with 25% acetonitrile in pH 3.5 phosphate buffer. The desired fractions showing retention time 3.8 min. (25% acetonitrile-buffer (pH 3.5)] were combined, concentrated, desalted and freeze-dried to give of the title compound ($I_C$): 7 mg (4.3%); Mp >200°C; IR $\nu_{max}$ (KBr) $cm^{-1}$ 1620; UV $\lambda_{max}$ (0.01N-NaOH) nm (ε) 504(12000); $^1$H NMR(400 MHz, DMSO-$d_6$) δ 1.17(3H, d, J=6.4 Hz, 5'-Me), 1.34(3H, d, J=7.3 Hz, 17-Me), 2.32(3H, s, 3-Me), 4.40(1H, q, J=7.3 Hz, 17-H), 4.49(1H, d, J=6.8 Hz, 1"-H), 4.58(2H, s, 5 & 6-H), 4.76(1H, d, J=8.1 Hz, 1'-H), 6.69(1H, d, J=2.1 Hz, 10-H), 7.20(1H, br s, 4-H), 7.26(1H, d, J=2.1 Hz, 12-H), 8.06(1H, br s, 7-H); FAB(+)-MS m/z 813(M+H).

Example 3:

Using similar procedures to those described in Example 2, 11-*O*-demethyl derivatives $I_D$-$I_F$ were prepared from pradimicin FA-1, BMS-181182 and BMS-181184, respectively, whose physico-chemical data are shown below.

11-*O*-Demethylpradimicin FA-1($I_D$): 2.6 mg (7.2%); Mp >200°C; IR $\nu_{max}$ (KBr) $cm^{-1}$ 1610; UV $\lambda_{max}$ (0.01N-NaOH) nm(ε) 502(13700); $^1$H NMR (400 MHz, DMSO-$d_6$) δ 2.25(3H, s, 3-Me), 2.42(3H, s, N-Me), 4.09(1H, q, J=7.3 Hz, 17-H), 4.38(1H, d, J=7.3 Hz, 1"-H), 4.39(2H, s, 5 & 6-H), 4.60(1H, d, J=7.3 Hz, 1'-H), 6.41(1H, br s, 10-H),6.91(1H, s, 4-H), 7.08(1H, br s, 12-H), 7.66(1H, s, 7-H); FAB(+)-MS m/z 843 (M+H), 865(M+Na).

11-*O*-Demethyl-BMS-181182($I_E$): 9 mg (5.0%); Mp >200°C; IR $\nu_{max}$ (KBr) $cm^{-1}$ 1610; UV $\lambda_{max}$ (0.01N-NaOH) nm(ε) 504(13700); $^1$H NMR(400 MHz, DMSO-$d_6$) δ 1.12(3H, d, J=6.4 Hz, 5'-Me), 2.24(3H, s, 3-Me), 2.58(6H, s, N-Me), 2.79 (1H, m, 4'-H) 3.03(1H, dd, J=8.6 & 7.3 Hz, 2"-H), 3.06(1H, t, J=10.7 Hz, 5"-$H_{ax}$), 3.14(1H, dd, J=9.0 & 8.6 Hz, 3"-H), 3.28(1H, m, 4"-H), 3.70 & 3.75 (1H each, dd, J=10.7 & 5.1 Hz, 17-$CH_2$), 3.97(1H, m, 17-H), 4.35(1H, d, J=11.1 Hz, 5-H), 4.40(1H, d, J=7.3 Hz, 1"-H), 4.41(1H, d, J=11.1 Hz, 6-H), 4.60(1H, d, J=7.7 Hz, 1'-H), 6.49(1H, d, J=2.1 Hz, 10-H), 6.90(1H, s, 4-H), 7.08(1H, d, J=2.1 Hz, 12-H), 7.73(1H, s, 7-H); FAB(+)-MS m/z 857 (M+H).

11-*O*-Demethyl-BMS-181184($I_F$): 2.9 mg (3.0%); Mp >200°C; IR $\nu_{max}$ (KBr) $cm^{-1}$ 1610; UV $\lambda_{max}$ (0.01N-NaOH) mn(ε) 504(14200); $^1$H NMR(400 MHz, DMSO-$d_6$) δ 1.11(3H, d, J=6.4 Hz, 5'-Me), 2.31(3H, s, 3-Me), 3.07(1H, t, J=11.1 Hz, 5"-$H_{ax}$), 3.12(1H, t, J=8.6 Hz, 2"-H), 3.14(1H, t, J=8.6 Hz, 3"-H), 3.29(1H, m, 4"-H), 3.54(1H, dd, J=9.8 & 2.6 Hz, 3'-H), *ca*3.6(2H, m, 4' & 5'-H), 3.70(1H, dd, J=11.1 & 5.1 Hz, 5"-$H_{eq}$), 3.71 & 3.76(1H each, dd, J=11.1 & 5.1 Hz, 17-$CH_2$), 4.40(1H, d, J=7.3 Hz, 1"-H), 4.45(1H, t, J=5.1 Hz, 17-H), 4.44(1H, d, J=10.3 Hz, 5-H), 4.49(1H, J=10.3 Hz, 6-H), 4.63(1H, d, J=7.7 Hz 1'-H), 6.55(1H, br s, 10-H), 7.03(1H, s, 4-H), 7.13(1H, br s, 12-H), 7.86(1H, s, 7-H);FAB(+)-MS m/z 837 (M+H), 852(M+Na).

Example 4: 11-*O*-Dexylosylpradimicin T1 methyl ester($A^1$)

To a suspension of 11-*O*-dexylosylpradimicin T1 (300 mg, 0.375 mmol) in MeOH (30 ml) was added thionyl chloride (96 μl, 1.32 mmol) and the mixture was stirred at room temperature overnight. After evaporation of the solvent, the residue was precipitated from MeOH-ether to give the title compound $A^1$ (316 mg, quantitative yield): $^1$H NMR(400 MHz, DMSO-$d_6$) δ 1.10(3H, d, J=6.4 Hz, 5'-Me), 2.34(3H, s, 3-Me), 3.06(1H, t, J=11.5 Hz, 5"-$H_{ax}$), 3.11(1H, t, J=8.6 Hz, 2"-H), 3.13(1H, t, J=8.6 Hz, 3"-H), 3.28(1H, m, 4"-H), 3.45(1H, m, 4'-H), 3.53(1H, m, 3'-H), 3.59(2H, m, 2'- & 5'-H), 3.68(3H, s, COOMe), 3.69(1H, dd, J=11.5 & 5.1 Hz, 5"$H_{eq}$), 3.99(2H, d, J=6.4 Hz, 17-$CH_2$), 4.40(1H, d, J=6.8 Hz, 1"-H), 4.48(1H, d, J=10.2 Hz, 5-H), 4.56(1H, d, 6-H), 4.63(1H, d, J=7.3 Hz, 1'-H), 6.65(1H, d, J=2.1 Hz, 10-H), 7.15(1H, s, 4-H), 7.22(1H, d, 12-H), 8.01(1H, s, 7-H); FAB(+)-MS m/z 814(M+H).

Example 5: 11-*O*-Dexylosyl-11-*O*-ethylpradimicin T1 ($II_A$)

Ethyl bromide (10 μl, 0.14 mmol) was added to a mixture of 11-*O*-dexylosylpradimicin T1 methyl ester $A^1$ (25 mg, 0.030 mmol; see example 4, above) and $K_2CO_3$ (8 mg, 0.058 mmol) in DMSO (0.3 ml) and the mixture was stirred overnight at room temperature. To the mixture was added 1N NaOH (3 ml) and the mixture was stirred overnight at room temperature. After acidification with diluted HCl, the mixture was chromatographed on a reversed phase column, which was washed with water and then eluted with 10-15% aqueous acetonitrile. The fractions containing the desired product were combined, concentrated *in vacuo* and freeze-dried to give 13 mg (52%) of the product $II_A$: Mp 200-210°C(dec.); UV $\lambda_{max}$ (0.01N-NaOH) nm (ε) 497 (14500); $^1$H NMR(400 MHz, DMSO-$d_6$) δ 1.12(3H, d, J=6.4 Hz, 5'-Me), 1.38(3H,t, *J*=7.3 Hz, $CH_2CH_3$),2.31 (3H, s, 3-Me), 3.85 (2H, d, *J*=6.4 Hz, 17-$CH_2$), 4.22 (2H, q, *J*=7.3 Hz, 11-*O*-$OCH_2$), 4.40 (1H, d, *J*=6.8 Hz, 1"-H), 4.42 (1H, d, *J*=11.7 Hz, 5-H), 4.49 (1H, br d, *J*=11.7 Hz, 6-H), 4.63 (1H, d, *J*=7.7 Hz, 1'-H), 6.75 (1H, d, *J*=2.6 Hz, 10-H), 7.00 (1H, s, 4-H), 7.16 (1H, d, *J*=2.6 Hz, 12-H), 7.82 (1H, s, 7-H); FAB(-)-MS m/z 827 (M).

Example 6:

Treatment of the ester of Example 4 ($A^1$) with 1-bromo-2-fluoroethane, allyl bromide, benzyl bromide, ethyl bromoacetate or iodacetamide, followed by deblocking and purification as described in Example 5 afforded compounds $II_B$-$II_F$, respectively.

11-*O*-Dexylosyl-11-*O*-(2-fluoroethyl)pradimicin T1 ($II_B$): 10 mg (39%); Mp 200-210°C(dec.); UV $\lambda_{max}$ (0.01N-NaOH) nm(ε) 497 (14700); $^1$H NMR (400 MHz, DMSO-$d_6$) δ 1.11 (3H, d, *J*=6.4 Hz, 5'-Me), 2.31 (3H, s, 3-Me), 3.90 (2H, d, *J*=6.0 Hz, 17-$CH_2$), 4.40 (1H, d, *J*=7.7 Hz, 1"-H), 4.64 (1H, d, *J*=7.3 Hz, 1'-H), 4.79 (2H, dt, *J*=47.4 & 3.4 Hz, $CH_2F$), 6.76 (1H, d, *J*=2.6 Hz, 10-H), 6.95 (1H, s, 4-H), 7.16 (1H, d, *J*=2.6 Hz 12-H), 7.74 (1H, s, 7-H); FAB(-)-MS m/z 845 (M).

11-*O*-Allyl-11-*O*-dexylosylpradimicin T1 ($II_C$): 7 mg (28%); Mp 220-230°C(dec); UV $\lambda_{max}$ (0.01N-NaOH) nm(ε) 497 (15200); $^1$H NMR (400 MHz, DMSO-$d_6$) δ 1.11 (3H, d, *J*=6.4 Hz, 5'-Me), 2.33 (3H, s, 3-Me), 3.91 (2H, d, *J*=6.0 Hz, 17-$CH_2$), 4.40 (1H, d, J=6.8 Hz, 1"-H), 4.45 (1H, d, *J*=10.7 Hz, 5-H), 4.54 (1H, d, *J*=10.7 Hz, 6-H), 4.63 (1H, d, *J*=7.3 Hz, 1'-H), 4.79 (2H, d, *J*=5.1 Hz, 11-$OCH_2$), 5.33 (1H, dd, *J*=10.7 &1.7 Hz, vinyl proton), 5.45 (1H, dd, J=16.1 & 1.7 Hz, vinyl proton), 6.08 (1H, m, vinyl proton), 6.83 (1H, br d, 10-H), 7.03 (1H, s, 4-H), 7.24 (1H, br d, 12-H), 7.87 (1H, s, 7-H); FAB(-)-MS m/z 839(M).

11-*O*-Benzyl-11-*O*-dexylosylpradimicin T1 ($II_D$): 10 mg (37%); Mp 200-210°C(dec); UV $\lambda_{max}$ (0.01N-NaOH) nm(ε) 497 (15100); $^1$H NMR (400 MHz, DMSO-$d_6$) δ 1.11 (3H, d, *J*=6.2 Hz, 5'-Me), 2.30 (3H, s, 3-Me), 3.90 (2H, d, *J*=6.4 Hz, 17-$CH_2$), 4.40 (1H, d, *J*=6.8 Hz, 1"-H), 4.42 (1H, d, *J*=10.3 Hz, 5-H), 4.47 (1H, d, *J*=10.3 Hz, 6-H), 4.63 (1H, d, *J*=7.3 Hz, 1'-H), 5.25 (2H, s, 11-$OCH_2$), 6.82 (1H, d, *J*=2.6 Hz, 10-H), 6.97 (1H, s, 4-H), 7.23 (1H, d, *J*=2.6 Hz, 12-H), 7.3 - 7.5 (5H, m, Ph), 7.77 (1H, s, 7-H), FAB(-)-MS m/z 889 (M).

11-*O*-Carboxymethyl-11-*O*-dexylosylpradimicin T1 ($II_E$): 17 mg (66%); Mp 210-220°C(dec.); UV $\lambda_{max}$ (0.01N-NaOH) nm(ε) 497 (14800); $^1$H NMR (400 MHz, DMSO-$d_6$) δ 1.11 (3H, d, *J*=6.4 Hz, 5'-Me), 2.32 (3H, s, 3-Me), 3.90 (2H, d, *J*=6.0 Hz, 17-$CH_2$), 4.40 (1H, d, *J*=6.8 Hz, 1"-H), 4.44 (1H, d, *J*=11.1 Hz, 5-H), 4.51, (1H, br d, *J*=11.1 Hz, 6-H), 4.64 (1H, d, *J*=8.1 Hz, 1'-H), 4.93 (2H, s, 11-$OCH_2$), 6.99 (1H, d, *J*=2.6 Hz, 10-H), 7.04 (1H, s, 4-H), 7.19 (1H, d, *J*=2.6 Hz, 12-H), 7.88 (1H, s, 7-H); FAB(+)- Ms m/z 858 (M+H).

11-*O*-Carbamoylmethyl-11-*O*-dexylosylpradimicin T1 ($II_F$): 11 mg (43%); Mp >230°C(dec.); UV $\lambda_{max}$ (0.01N-NaOH) nm (ε), 496 (15100); $^1$H NMR (400 MHz, DMSO-$d_6$) δ 1.11 (3H, d, *J*=6.4 Hz, 5'-Me), 2.33 (3H, s, 3-Me), 3.90 (2H, d, *J*=6.0 Hz, 17-$CH_2$), 4.40 (1H, d, *J*=7.3 Hz, 1"-H), 4.46 (1H, d, *J*=10.3 Hz, 5-H), 4.55 (1H, br d, *J*=10.3 Hz, 6-H), 4.64 (1H, d, *J*=7.7 Hz, 1'-H), 4.96 (2H, s, 11-$OCH_2$), 6.87 (1H, d, *J*=2.6 Hz, 10-H), 7.11 (1H, s, 4-H), 7.25 (1H, d, *J*=2.6 Hz, 12-H), 7.97 (1H, s, 7-H); FAB(-)-Ms m/z 856 (M).

Example 7: 4'-N-Cbz-11-*O*-demethylpradimicin A methyl ester ($B^1$) and 4'-N-Cbz-11-*O*-demethylpradimicin B methyl ester ($C^1$)

To a suspension of a mixture of $I_A$ and $I_B$ (1:1, 60 mg) in dry dichloromethane was added trimethylsilyl chloride (0.1 ml) and the mixture was stirred at room temperature for 10 minutes. Then N,O-bistrimethylsilylacetamide (0.4 ml) was added and the mixture was stirred at room temperature for 2 hours. To the clear solution was added carbobenzoxy chloride (0.1 ml) and the mixture was stirred at room temperature for 20 hours. After evaporating to dryness, the residue was dissolved in MeOH (6 ml). To the solution was added 1N HCl (3 ml) and the mixture was stirred at room temperature for one hour and then purified on a reversed phase column by eluting with 15 and 20% acetonitrile in pH 7.0 phosphate buffer. The fractions containing the desired products were collected, concentrated, desalted and freeze-dried to give 50 mg of a mixture of the 4'-N-Cbz derivative of $I_A$ and $I_B$ (1:1). The sample obtained above was treated with thionyl chloride (50 μl) in MeOH (6 ml) and the mixture was stirred at room temperature for 16 hours. After evaporating to dryness, the oily residue was chromatographed on a silica gel column (Wako-gel C-200, 15 g) eluting with chloroform-methanol (10:1-4:1). The fractions eluted with chloroform-methanol (10:1) were evaporated to dryness to give 21 mg of compound $C^1$: FAB(-)-MS m/z 842 (M). The fractions eluted with chloroform-methanol (4:1) were combined, evaporated and purified on a reverse phase column by eluting with 50% acetonitrile in pH 3.5 phosphate buffer. The desired fractions were collected, concentrated, desalted and freeze-dried to give 19 mg of compound $B^1$. FAB-(-)-MS m/z 974 (M).

Example 8: 11-Demethoxypradimicin A ($II_G$)

A mixture of compound $B^1$ (17 mg, 0.018 mmol), N-phenytrifluoromethanesulfonimide (9.4 mg, 0.026 mmol) and N, N-dimethylaminopyridine (3.2 mg, 0.026 mmol) in dry pyridine (1.7 ml) was stirred at room temperature for one hour. The mixture was diluted with pH 3.5 phosphate buffer (20 ml) and purified on a reversed phase column by eluting with 50 and 60% acetonitrile in pH 3.5 phosphate buffer. The desired fractions were

combined, concentrated, desalted and freeze-dried to give 16 mg (82.5%) of the 11-*O*-triflate derivative of compound $B^1$: FAB (+)-MS m/z 1107 (M+H), 1129(M+Na).

To a solution of the triflate (23 mg, 0.02 mmol) in dry DMF (2 ml) was sequentially added palladium acetate (9 mg, 0.04 mmol), triphenylphosphine (21 mg, 0.08 mmol), triethylamine (28 μl, 0.2 mmol) and 98% formic acid (8 μl, 0.21 mmol) and the mixture was stirred at room temperature for 4 hours. The mixture was diluted with pH 3.5 phosphate buffer and charged on a reversed phase column, which was washed with 20 and 30% acetonitrile in pH 3.5 phosphate buffer and then eluted with 40 and 50% acetonitrile in pH 3.5 phosphate buffer. Upon monitoring with HPLC, the desired fractions eluted with 50% acetonitrile in pH 3.5 phosphate buffer were combined, concentrated, desalted and freeze-dried to give 14 mg (73%) of the 11-deoxy derivative of compound $B^1$: Mp >200°C; FAB (+)-MS m/z 959 (M+H), 981 (M+Na).

A mixture of 11-deoxy derivative of compound $B^1$ (14 mg, 0.015 mmol) and 10% palladium on carbon (10 mg) in a mixture of MeOH (3 ml), water (1 ml) and acetic acid (1 ml) was hydrogenated at room temperature for 3 hours under hydrogen atmosphere. The catalyst was removed by filtration and the filtrate was charged on a reversed phase column, which was washed with water and eluted with 75% aqueous dioxane. The desired fractions were concentrated, diluted with MeOH (5 ml) and then treated with 1N NaOH (lml). The mixture was stirred at room temperature for 30 minutes, adjusted to pH 6 with 1N HCl and purified on a reversed phase column by eluting with 30% acetonitrile in pH 3.5 phosphate buffer. The desired fractions were combined, concentrated, desalted and freeze-dried to give 4 mg (34%) of compound $II_G$: Mp >200°C; UV $\lambda_{max}$ (0.01N-NaOH) nm(ε) 500 (11800); $^1H$ NMR (400 MHz, DMSO-$d_6$) δ 1.25 (3H, d, J=6.8 Hz, 5'-Me), 1.35 (3H, d, J=7.3 Hz, 17-Me), 2.30 (3H, s, 3-Me), 2.60 (3H, s, 4'-NMe), 3.11 (1H, t, J=11.1 Hz, 5"-$H_{ax}$), 3.12 (1H, m, 2"-H), 3.16 (1H, t, J=9.0 Hz, 3"-H), 3.3 (1H, m, 4"-H), 3.49 (1H, m, 4'-H), 3.51 (1H, t, J=8.1 Hz, 2'-H), 3.74 (1H, dd, J=11.1 & 5.1 Hz, 5"-$H_{eq}$), 3.85 (1H, m, 5'-H), 3.89 (1H, dd, J=9.8 & 4.7 Hz, 3'-H), 4.38 (1H, q, J=7.3 Hz, 17-H), 4.43 (1H, d, J=7.3 Hz, 1"-H), 4.49 (2H, s, 5 & 6-H), 4.75 (1H, d, J=8.1 Hz, 1'-H), 6.85 (1H, s, 4-H), 7.21 (1H, dd, J=8.1 & 0.9 Hz, 10-H), 7.62 (1H, dd, J=7.7 & 0.9 Hz, 12-H), 7.71 (1H, s, 7-H), 7.73 (1H, t, J=7.7 Hz, 11-H). FAB(+)-MS m/z 811 (M+H).

Example 9: 11-Demethoxypradimicin B ($II_H$)

By the similar procedures for the preparation of compound $II_G$, compound $C^1$(17 mg) was converted to the title compound $II_H$: 1.5 mg (26%); Mp >200°C; UV $\lambda_{max}$ (0.01N-NaOH) nm (ε) 502 (10400); $^1H$ NMR (400 MH, DMSO-$d_6$) δ 1.24 (3H, d, J=6.8 Hz, 5'-Me), 1.34 (3H, d, J=7.3 Hz, 17-Me), 2.27 (3H, s, 3-Me), 2.59 (3H, s, 4'-NMe), 3.68 (1H, m, 3'-H), 3.79 (1H, m, 5'-H), 4.36 (1H, q, J=7.3 Hz, 17-H), 4.43 (1H, d, J=10.3 Hz, 5-H), 4.47 (1H, d, J=10.3 Hz, 6-H), 4.64 (1H, d, J=8.1 Hz, 1'-H), 6.88 (1H, s, 4-H), 7.20 (1H, dd, J=8.1 & 0.9 Hz, 10-H), 7.62 (1H, dd, J=7.7 & 0.9 Hz, 12-H), 7.71 (1H, s, 7-H), 7.72 (1H, dd, J=8.1 & 7.7 Hz, 11-H); FAB(+)-MS m/z 679 (M+H), 701 (M+Na).

Example 10: 11-*O*-Dexylosyl-11-*O*-trifluoromethanesulfonylpradimicin T1 Methyl ester ($D^1$)

To a solution of 11-*O*-dexylosylpradimicin T1 methyl ester ($A^1$, 157 mg. 0.193 mmol) in dry pyridine (8 ml) were added dimethylaminopyridine (35 mg, 0.290 mmol) and N-phenyltrifluoromethanesulfonimide (103 mg, 0.290 mmol) and the mixture was stirred at room temperature for 1 hour. After being diluted with pH 3.5 buffer, the mixture was charged on a $C_{18}$ column, which was washed with water and then eluted with 40 and 45% acetonitrile-buffer (pH 3.5). The fractions containing the desired product were evaporated, desalted and lyophilized to afford compound $D^1$ (155 mg, 85%): $^1H$ NMR(400MHz, (DMSO-$d_6$) δ 1.10(3H, d, J=6.0 Hz, 5'-Me), 2.34(3H, s, 3-Me), 3.06(1H, t, J=10.7 Hz, 5"-$H_{ax}$), 3.11(1H, t, J=8.1 Hz, 2"-H), 3.13(1H, t, J=8.1 Hz, 3"-H), 3.28(1H, m, 4"-H), 3.46(1H, m, 4'-H), 3.52(1H, m, 3'-H), 3.60(2H, m, 2'- & 5'-H), 3.68(3H, s, COOMe), 3.70(1H, dd, J=11.5 & 5.6 Hz, 5"-$H_{eq}$), 3.99(2H, d, J=6.0 Hz, 17-$CH_2$), 4.40(1H, d, J=6.8 Hz, 1"-H), 4.49(1H, d, J=9.8 Hz, 5-H), 4.58(1H, d, 6-H), 4.64(1H, d, J=7.3 Hz, 1'-H), 7.16(1H, s, 4-H), 7.66(1H, d, J=2.6 Hz, 10-H), 7.73(1H, d, 12-H), 8.03(1H, s, 7-H); FAB(+)-MS m/z 946(M+H).

Example 11: 11-Dexylosyloxypradimicin T1 ($II_I$)

To a solution of compound $D^1$ (43 mg, 0.046 mmol) in DMF (2 ml) were sequentially added Pd $(OAc)_2$(10.2 mg, 0.046 mmol), $PPh_3$(24 mg, 0.091 mmol), triethylamine (63.4 μl, 0.455 mmol) and 98% formic acid (10.3 μl, 0.273 mmol) and the whole mixture was stirred at room temperature for 2 hours. After diluting with water, the mixture was charged on a $C_{18}$ column (50 ml), which was washed with water and then eluted with 30% acetonitrile-buffer (pH 3.5). The fractions containing the desired product were evaporated, desalted and lyophilized to afford the methyl ester of title compound (36 mg, quantitative): $^1H$ NMR(400 MHz, DMSO-$d_6$) δ

1.10(3H, d, J=6.4 Hz, 5'-Me), 2.33(3H, s, 3-Me), 3.09(1H, t, J=11.1 Hz, 5"-$H_{ax}$), 3.11(1H, t, J=8.6 Hz, 2"-H), 3.13(1H, t, J=8.6 Hz, 3"-H), 3.28(1H, m, 4"-H), 3.43 (1H, m, 4'-H), 3.53(1H, m, 3'-H), 3.68(3H, s, COOMe), 3.70(1H, dd, J=11.1 & 5.1 Hz, 5"-$H_{eq}$), 3.99(2H, ABq, J=16.0 Hz, 17-$CH_2$), 4.40(1H, d, J=7.3 Hz, 1"-H), 4.47(1H, d, J=11.1 Hz, 5-H), 4.55(1H, d, 6-H), 4.64(1H, d, J=7.3 Hz, 1'-H), 7.10(1H, s, 4-H), 7.37(1H, brd, J=7.7 Hz, 10-H), 7.50(1H, d, J=7.7 Hz, 12-H), 7.81(1H, t, J=7.7 Hz, 11-H), 7.95(1H, s, 7-H); FAB(+)-MS m/z 798(M+H).

A solution of the above methyl ester (36 mg, 0.045 mmol) in MeOH (1 ml) was treated with 1N-NaOH (0.1 ml) at room temperature for 1 hour. After evaporation of the solvent, the residue was charged on a $C_{18}$ column (50 ml), which was washed with water and then eluted with 13% acetonitrile-buffer (pH 7.0). The fractions containing the desired product were evaporated, desalted and lyophilized to afford the title compound $II_I$ (5 mg, 14% from $D^1$): Mp>230°C; IR $\nu_{max}$(KBr) $cm^{-1}$ 3400, 1720, 1620; UV $\lambda_{max}$(0.01N-NaOH) nm ($\varepsilon$) 499 (13000); $^1H$ NMR(400 MHz, DMSO-$d_6$) $\delta$ 1.12(3H, d, J=6.4 Hz, 5'-Me), 2.28(3H, s, 3-Me), 3,08(1H, t, J=11.1 Hz, 5"-$H_{ax}$), 3.10(1H, t, J=9.0 Hz, 2"-H), 3.15(1H, t, J=9.0 Hz, 3"-H), 3.29(1H, m, 4"-H), 3.55(1H, m, 3'-H), 3.61(1H, q, J=6.4 Hz, 5'-H), 3.70(1H, dd, J=11.1 & 5.5 Hz, 5"-$H_{eq}$), 3.71(1H, t, J=7.7 Hz, 2'-H), 3.86 & 3.91(2H, ABq, J=17.1 Hz, 17-$CH_2$), 4.40(1H, d, J=10.7 Hz, 5-H), 4.41(1H, d, J=8.1 Hz, 1"-H), 4.45(1H, d, 6-H), 4.64(1H, d, J=7.7 Hz, 1'-H), 6.96(1H, s, 4-H), 7.21(1H, d, J=8.1 Hz, 10-H), 7.61(1H, d, J=7.7 Hz, 12-H), 7.71(1H, dd, J=7.7 & 8.1 Hz, 11-H), 7.76(1H, s, 7-H); FAB(+)-MS m/z 806(M+Na).

Example 12: 11-Dexylosyloxy-11-dimethylaminopradimicin T1 ($II_J$)

To a solution of compound $D^1$ (19 mg, 0.020 mmol) in DMF (1.0 ml) was added a 2M solution of dimethylamine in EtOAc[0.4 ml; prepared from $Me_2NH$-HCl (164 mg, 2.0 mmol) by treating with excess NaOH in water (1 ml), followed by trapping gaseous $Me_2NH$ in upper EtOAc layer] and the mixture was stirred at room temperature overnight. After being poured into pH 3.5 buffer, the mixture was charged on a $C_{18}$ column, which was washed with water and then eluted with 40% acetonitrile-buffer (pH 3.5). The appropriate fractions were evaporated, desalted and lyophilized to afford the methyl ester of title compound (10mg). The sample in MeOH (2 ml) was treated with 1N-NaOH (0.5 ml) at room temperature for 1 hour. After evaporation of the solvent, the residue was charged on a $C_{18}$ column, which was washed with water and then eluted with 15% acetonitrile-buffer (pH 7.0). The desired fractions were evaporated, desalted and lyophilized to afford the title compound $II_J$ (6 mg, 34% from $D^1$): Mp>230°C; IR $\nu_{max}$ (KBr) $cm^{-1}$ 3400, 1730, 1600; UV $\lambda_{max}$ (0.01N-NaOH) nm($\varepsilon$) 522(12600); $^1H$ NMR(400 MHz, DMSO-$d_6$) $\delta$ 1.12(3H, d, J=6.4 Hz, 5'-Me), 2.30(3H, s, 3-Me), 3.07(1H, t, J=11.5 Hz, 5"-$H_{ax}$), 3.11(6H, s, $NMe_2$), 3.12(1H, t, J=9.0 Hz, 2"-H),3.14(1H, t, J=9.0 Hz, 3"-H), 3.70(1H, dd, J=11.5 & 5.1 Hz, 5"-$H_{eq}$), 3.71(1H, q, J=6.4 Hz, 5'-H), 3.90(2H, d, J=6.0 Hz, 17-$CH_2$), 4.38(1H, d, J=10.7 Hz, 5-H), 4.41(1H, d, J=6.8 Hz, 1"-H), 4.45(1H, d, 6-H); 4.63(1H, d, J=7.7 Hz, 1'-H), 6.27(1H, brd, 10-H), 6.97(1H, s, 4-H), 702(1H, brd, 12-H), 7.76(1H, s, 7-H); FAB(+)-MS m/z 827(M+H).

Example 13: 11-Carbamoyl ($II_K$) and 11-Cyano($II_L$)-11- dexylosyloxy pradimicin T1

To a solution of compound $D^1$ (60 mg, 0.063 mmol) in DMF (4 ml) were sequentially added $Pd(OAc)_2$ (28 mg, 0.126 mmol), $PPh_3$ (66 mg, 0.254 mmol) and tributyltin cyanide (200 mg, 0.64 mmol) and the whole mixture was heated at 70°C for 3 days. The solution was diluted with MeOH (20 ml) and treated with 1N-NaOH (5 ml) at room temperature for 1 hour. After evaporation of the solvent, the residue was charged on a $C_{18}$ column (50 ml), which was washed with water and then eluted with 5, 10 and 15% aqueous acetonitrile to give the following two fractions after evaporation and lyophilization.

Fraction 1; 5 mg (9.5% from $D^1$) of $II_K$: Retention Time 1.9 min [30% acetonitrile-buffer (pH 3.5)]; Mp>230°C; IR $\nu_{max}$ (KBr) $cm^{-1}$ 3400, 1720, 1620; UV $\lambda_{max}$ (0.01N-NaOH) nm ($\varepsilon$) 508 (11800); $^1H$ NMR(400 MHz, DMSO-$d_6$) $\delta$ 1.11(3H, d, J=6.4 Hz, 5'-Me), 2.29(3H, s, 3-Me), 3.07(1H, t, J=10.7 Hz, 5"-$H_{ax}$), 3.10(1H, t, J=7.3 Hz, 2"-H), 3.14(1H, dd, J=7.3 & 9.0 Hz, 3"-H), 3.29(1H, m, 4"-H), 3.54(1H, dd, J=2.6 & 9.8 Hz, 3'-H), 3.59(1H, d, J=2.6 Hz, 4'-H), 3.70(1H, dd, J=10.7 & 5.1 Hz, 5"-$H_{eq}$), 3.71(1H, t, J=7.7 Hz, 2'-H), 3.89(2H, d, J=6.0 Hz, 17-$CH_2$), 4.40(1H, d, J=10.3 Hz, 5-H), 4.40(1H, d, J=7.3 Hz, 1"-H), 4.46(1H, d, 6-H), 4.64(1H, d, J=7.7 Hz, 1'-H), 6.95(1H, s, 4-H), 7.20(2H, br s, $CONH_2$), 7.62(1H, d, J=1.3 Hz, 10-H), 7.76(1H, s, 7-H), 8.09(1H, d, J=1.3 Hz, 12-H); FAB(+)-MS m/z 826(M).

Fraction 2; 3 mg (5.8% from $D^1$) of $II_L$: Retention Time 5.9 min [same as above]; Mp>230°C; IR $\nu_{max}$ (KBr) $cm^{-1}$ 3400, 1720, 1620; UV $\lambda_{max}$ (0.01N-NaOH) nm($\varepsilon$) 506 (13200); $^1H$ NMR(400 MHz, DMSO-$d_6$) $\delta$ 1.11(3H, d, J=6.4 Hz, 5'-Me), 2.30(3H, s, 3-Me), 3.07(1H, t, J=10.7 Hz, 5"-$H_{ax}$), 3.10(1H, t, J=7.3 Hz, 2"- H), 3.14(1H, dd, J=7.3 & 8.6 Hz, 3"-H), 3.29(1H, m, 4"-H), 3.54(1H, dd, J=3.4 & 9.8 Hz, 3'-H), 3.59(1H, d, J=3.4 Hz, 4'-H), 3.70(1H, dd, J=11.1 & 5.1 Hz, 5"-$H_{eq}$), 3.70(1H, t, J=7.7 Hz, 2'-H), 3.90(2H, d, J=5.6 Hz, 17-$CH_2$), 4.40(1H, d, J=7.3 Hz, 1"-H), 4.41 (1H, d, J=11.5 Hz, 5-H), 4.47(1H, d, 6-H), 4.63(1H, d, J=7.7 Hz, 1'-H), 6.95(1H, s, 4-H), 7.72(1H, d, J=1.7 Hz, 10-H), 7.74(1H, s, 7-H), 7.87(1H, d, J=1.7 Hz, 12-H); FAB(+)-MS mz 809(M+H).

Reasonable variations, such as those which would occur to a skilled artisan, can be made herein without departing from the scope of the invention.

## Claims

1. A compound of structure I:

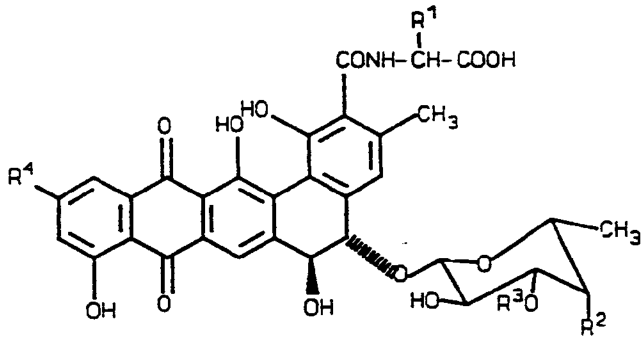

(I)

wherein

$R^1$ is H, $CH_3$, or a $CH_2OH$;
$R^2$ is OH, or $NR^5R^6$
$R^3$ is H or

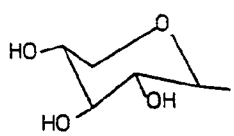

(β-D-xylose)

$R^5$ and $R^6$ are H or $CH_3$;
$R^4$ is H, OH, $OR^7$, $OCH_2R^8$, $OSO_2CF_3$ $N(CH_3)_2$, CN, $OCONH_2$, or CN;
$R^7$ is $C_{2-5}$ alkyl, $C_{2-5}$ alkenyl, or $C_{2-5}$ haloalkyl (halo = Cl or F); and
$R^8$ is phenyl, COOH or $CONH_2$, with the proviso that when $R^4$ is OH, $R^1$ is not H.

2. The acid or base addition products of the compound of claim 1.

3. A pharmaceutical composition comprising an effective antibiotic amount of at least one compound of claim 1 and one or more pharmaceutically acceptable excipients.

4. A process for treating a patient suffering from an infectious disease comprising the step of administering to said patient an antibiotic effective amount of at least one compound of claim 1.

5. The compound of claim 1 wherein $R^4$ is OH.

6. The compound of claim 5 wherein $R^1$ is $CH_3$, $R^2$ is $NHCH_3$ and $R^3$ is β-D-xylose.

7. The compound of claim 5 wherein $R^1$ is $CH_3$, $R^2$ is $NHCH_3$ and $R^3$ is H.

8. The compound of claim 5 wherein $R^1$ is $CH_3$, $R^2$ is $NH_2$ and $R^3$ is β-D-xylose.

9. The compound of claim 5 wherein $R^1$ is $CH_2OH$, $R^2$ is $NHCH_3$ and $R^3$ is β-D-xylose.

10. The compound of claim 5 wherein $R^1$ is $CH_2OH$, $R^2$ is $N(CH_3)_2$ and $R^3$ is β-D-xylose.

11. The compound of claim 5 wherein $R^1$ is $CH_2OH$, $R^2$ is OH and $R^3$ is β-D-xylose.

**12.** A process for the preparation of 11-hydroxy pradimicin derivatives comprising the steps:
(1) Contacting an 11-methoxy pradicin with an aryl ether cleavage reagent while heating, and
(2) isolating the 11-hydroxy product.

**13.** The process of claim 12 wherein steps (1) and (2) take place in the presence of a solvent selected from the group consisting of: collidine, lutidine, dimethylformamide and mixtures thereof.

**14.** The process of claim 12 wherein step (2) is carried out via elution with a buffer on a reversed phase column.

**15.** A compound of structure II:

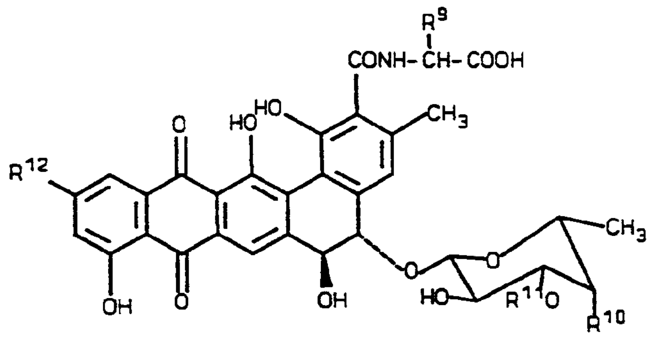

(II)

wherein
$R^9$ is H or $CH_3$;
$R^{10}$ is OH or $NHCH_3$;
$R^{11}$ is H or β-D-xylose, and
$R^{12}$ is H, $OC_2H_5$, $OCH_2CH_2F$, $OCH_2CH=CH_2$, $OCH_2$phenyl, ($OCH_2Ph$), $OCH_2COOH$, $OCH_2CONH_2$, $N(CH)_2$, $CONH_2$, or CN,

**16.** The compound of claim 12 wherein $R^9$ is H, $R^{10}$ is OH, and $R^{11}$ is β-D-xylose.

**17.** The compound of claim 13 wherein $R^{12}$ is $OC_2H5$.

**18.** The compound of claim 13 wherein $R^{12}$ is $OCH_2CH_2F$.

**19.** The compound of claim 13 wherein $R^{12}$ is $OCH_2CH=CH_2$.

**20.** The compound of claim 13 wherein $R^{12}$ is $OCH_2$phenyl.

**21.** The compound of claim 13 wherein $R^{12}$ is $OCH_2COOH$.

**22.** The compound of claim 13 wherein $R^{12}$ is $OCH_2CONH_2$.

**23.** The compound of claim 13 wherein $R^{12}$ is H.

**24.** The compound of claim 13 wherein $R^{12}$ is $N(CH_3)_2$.

**25.** The compound of claim 13 wherein $R^{12}$ is $CONH_2$.

**26.** The compound of claim 13 wherein $R^{12}$ is CN.

**27.** The compound of claim 12 wherein $R^9$ is $CH_3$, $R^{10}$ is $NHCH_3$, and $R^{12}$ is H.

**28.** The compound of claim 24 wherein $R^{11}$ is β-D-xylose.

**29.** The compound of claim 24 wherein $R^{11}$ is H.

**30.** A process for the production of compounds of formula II:

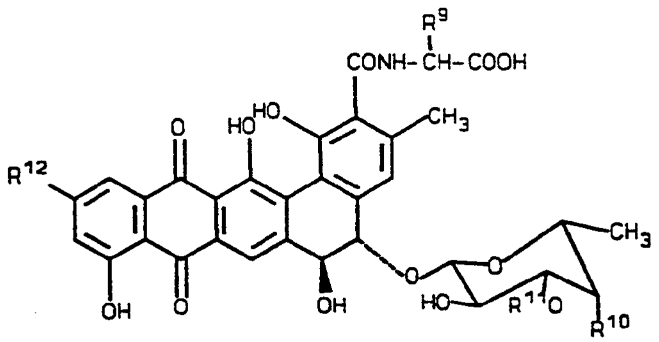

(II)

wherein

$R^9$ is H or $CH_3$;

$R^{10}$ is OH or $NHCH_3$;

$R^{11}$ is H or β-D-xylose, and

$R^{12}$ is H, $OC_2H_5$, $OCH_2CH_2F$, $OCH_2CH{=}CH_2$, $OCH_2$phenyl, ($OCH_2Ph$), $OCH_2COOH$, $OCH_2CONH_2$, $N(CH)_2$, $CONH_2$, or CN,

comprising the steps of:

(1) contacting an appropriate 11-hydroxy pradimicin with a trifluoromethane-sulfonylation reagent under basic conditions;

(2) contacting the product of step (1) with hydride ion and a catalyst;

(3) deblocking the carboxy and amino protective groups from the step (3) product using alkaline hydrolysis; and

(4) isolation of the compounds of formula II.

**31.** The process of claim 30 wherein step (1) takes place in the presence of a basic organic solvent and a triflating amount of N-phenyltriufluoromethanesulfonimide or trifluoromethanesulfonic anhydride.

**32.** The process of claim 31 wherein the product of step (1) is purified via reverse phase column chromatography prior to step (2).

**33.** The process of claim 30 wherein the nucleophilic reagent used in step (2) is a nucleophilic base or a trialkyl substituted metal reagent.

**34.** The process of claim 31 wherein hydride reduction occurs during step (3).

European Patent Office

## PARTIAL EUROPEAN SEARCH REPORT

which under Rule 45 of the European Patent Convention shall be considered, for the purposes of subsequent proceedings, as the European search report

Application Number: EP 93 40 2886

### DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X | THE JOURNAL OF ANTIBIOTICS vol. 46, no. 4 , April 1993 , TOKYO, JP pages 598 - 605 T. HASEGAWA ET AL 'Pradimicins T1 and T2, new antifungal antibiotics produced by an actinomycete. II. Structures and biosynthesis.' * page 599 - page 601 * | 1-5 | C07H15/244<br>A61K31/71 |
| A | EP-A-0 388 982 (BRISTOL-MYERS SQUIBB COMPANY) * the whole document * | 1-4,12, 15,30 | |
| A | EP-A-0 420 552 (ZAIDAN HOJIN BISEIBUTSU) * the whole document * | 1-4,12, 15,30 | |
| A | EP-A-0 368 349 (BRISTOL-MYERS SQUIBB COMPANY) * the whole document * | 1-4,12, 15,30 | |
| | | | TECHNICAL FIELDS SEARCHED (Int.Cl.5) |
| | | | C07H<br>A61K |

### INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 15 March 1994 | Moreno, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 93 40 2886
-C-

Remark: Although claim 4 is directed to a method of treatment of the human/animal body (Art. 52(4) EPC) the search has been carried out and based on the alleged effects of the compound/composition